(19) Europäisches Patentamt
European Patent Office
Office européen des brevets

(11) **EP 3 936 539 A1**

(12) **EUROPEAN PATENT APPLICATION**
published in accordance with Art. 153(4) EPC

(43) Date of publication:
**12.01.2022 Bulletin 2022/02**

(51) Int Cl.:
**C08F 20/06** (2006.01)     **A61F 13/53** (2006.01)
**B01J 20/26** (2006.01)     **B01J 20/28** (2006.01)
**B01J 20/30** (2006.01)

(21) Application number: **20769793.9**

(22) Date of filing: **05.03.2020**

(86) International application number:
**PCT/JP2020/009478**

(87) International publication number:
**WO 2020/184388 (17.09.2020 Gazette 2020/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.03.2019   JP 2019042879**

(71) Applicant: **Sumitomo Seika Chemicals Co., Ltd.
Kako-gun, Hyogo 675-0145 (JP)**

(72) Inventor: **NISHIDA, Moe
Himeji-shi, Hyogo 672-8076 (JP)**

(74) Representative: **Nederlandsch Octrooibureau
P.O. Box 29720
2502 LS The Hague (NL)**

(54) **WATER-ABSORBING RESIN PARTICLES, WATER-ABSORBING ARTICLE, METHOD FOR PRODUCING WATER-ABSORBING RESIN PARTICLES, AND METHOD FOR INCREASING ABSORBED AMOUNT OF ABSORBER UNDER PRESSURE**

(57)     Disclosed are water-absorbing resin particles 10a in which an expansion retention rate calculated by the formula: expansion retention rate (%) = $(V_1 / V_0) \times 100$ is 98% or more. $V_0$ is the volume of the swollen gel formed when $1.000 \pm 0.001$ g of the water-absorbing resin particles 10a absorb $20.0 \pm 0.1$ g of pure water. $V_1$ is the volume of the swollen gel formed when $1.000 \pm 0.001$ g of the water-absorbing resin particles 10a absorb $20.0 \pm 0.1$ g of physiological saline.

**EP 3 936 539 A1**

## Description

### Technical Field

The present invention relates to a water-absorbing resin particle, an absorbent article, a method for producing a water-absorbing resin particle, and a method for increasing the absorption amount of an absorber under pressure.

### Background Art

Conventionally, an absorber containing water-absorbing resin particles has been used in an absorbent article for absorbing a liquid containing water as a main component, such as urine. For example,

Patent Document 1 discloses an absorber which is made thin under pressure by integrating and molding absorbing fibers such as pulp and a high-absorbing polymer having a specific gel elastic modulus and an artificial urine absorption amount under a pressure of 20 g/cm$^2$ while defibrating and mixing them by an air-laying method.

### Citation List

### Patent Literature

[Patent Document 1] Japanese Unexamined Patent Application Publication No. 2002-172139

## Summary of Invention

### Technical Problem

An aspect of the present invention provides a water-absorbing resin particle capable of increasing the absorption amount of an absorber under pressure.

### Solution to Problem

An aspect of the present invention relates to water-absorbing resin particles, an expansion retention rate of which is 98% or more. The expansion retention rate is calculated by the formula: expansion retention rate (%) = ($V_1$ / $V_0$) $\times$ 100. $V_0$ is the volume of the swollen gel formed when 1.000 $\pm$ 0.001 g of the water-absorbing resin particles absorb 20.0 $\pm$ 0.1 g of pure water. $V_1$ is the volume of the swollen gel formed when 1.000 $\pm$ 0.001 g of the water-absorbing resin particles absorb 20.0 $\pm$ 0.1 g of physiological saline.

The absorber containing the water-absorbing resin particles having an expansion retention rate of 98% or more can exhibit a high absorption amount under pressure. In general, the volume of the swollen gel formed by water absorption tends to be smaller when the water-absorbing resin particles absorb physiological saline than when the water-absorbing resin particles absorb pure water. This is considered to be due to the influence of the difference in osmotic pressure generated when the water-absorbing resin particles absorb liquid. The expansion retention rate of 98% or more of the water-absorbing resin particles means that the volume $V_1$ of the swollen gel formed when the water-absorbing resin particles absorb physiological saline is substantially equal to or more than the volume $V_0$ of the swollen gel formed when the water-absorbing resin particles absorb pure water. When the hardness of the individual water-absorbing resin particles is uniform, the volume of the swollen gel is small even in pure water having a high osmotic pressure, and a difference in the volume of the swollen gel between pure water and physiological saline is less likely to occur. That is, it is considered that the expansion retention rate of 98% or more reflects that the hardness of the individual water-absorbing resin particles is uniform when the water-absorbing resin particles absorb water. The present inventor presumes that, when the hardness of the particles is uniform, the particles are less likely to be affected by pressure, and therefore, the absorber containing the water-absorbing resin particles having the expansion retention rate of 98% or more can exhibit a large absorption amount even under pressure.

Another aspect of the present invention relates to an absorbent article including a liquid impermeable sheet, an absorber, and a liquid permeable sheet, wherein the liquid impermeable sheet, the absorber, and the liquid permeable sheet are disposed in this order. The absorber contains the water-absorbing resin particles.

Still another aspect of the present invention relates to a method for producing water-absorbing resin particles, including selecting water-absorbing resin particles, the expansion retention rate of which is 98% or more.

Still another aspect of the present invention relates to a method for increasing the absorption amount under pressure of an absorber containing water-absorbing resin particles. The method includes increasing the expansion retention rate of the water-absorbing resin particles.

## Advantageous Effects of Invention

According to one aspect of the present invention, there is provided water-absorbing resin particles capable of increasing the absorption amount of an absorber under pressure.

## Brief Description of Drawings

FIG. 1 is a cross-sectional view showing an embodiment of an absorbent article.
FIG. 2 is a plan view showing an example of a stirring blade (a flat blade having a slit in a flat plate portion).
FIG. 3 is a schematic view showing a method for measuring the water absorption capacity under load of water-absorbing resin particles.
FIG. 4 is a schematic view showing a method for measuring the non-pressurization DW of water-absorbing resin particles.
FIG. 5 is a schematic view showing a method for measuring the absorption amount under pressure of the absorber.
FIG. 6 is a schematic view showing a method for measuring the absorption amount under pressure of the absorber.

## Description of Embodiments

Hereinafter, some embodiments of the present invention will be described in detail. However, the present invention is not limited to the following embodiments.

In the present specification, "acrylic" and "methacrylic" are collectively referred to as "(meth) acrylic". Similarly, "acrylate" and "methacrylate" are also referred to as "(meth) acrylate". "(Poly)" shall mean both with and without the prefix "poly". In the numerical ranges described stepwise in the present specification, the upper limit value or the lower limit value of the numerical range of a certain step can be arbitrarily combined with the upper limit value or the lower limit value of the numerical range of another step. In the numerical ranges described in the present specification, the upper limit value or the lower limit value of the numerical range may be replaced with the values shown in the examples. "A or B" means either A or B or both A and B. The term "water-soluble" means that it exhibits a solubility in water of 5% by mass or more at 25°C. The materials exemplified in the present specification may be used alone or in combination of two or more. When a plurality of substances corresponding to each component are present in the composition, the content of each component in the composition means the total amount of the plurality of substances present in the composition unless otherwise specified.

The water-absorbing resin particles according to an embodiment exhibit an expansion retention rate of 98% or more. The expansion retention rate is calculated by the formula: expansion retention rate (%) = $(V_1 / V_0) \times 100$. In the formula, $V_0$ is the volume of the swollen gel formed when $1.000 \pm 0.001$ g of the water-absorbing resin particles absorb $20.0 \pm 0.1$ g of pure water, and $V_1$ is the volume of the swollen gel formed when $1.000 \pm 0.001$ g of the water-absorbing resin particles absorb $20.0 \pm 0.1$ g of physiological saline. The volumes $V_0$ and $V_1$ of the swollen gel are typically measured in an environment of $25 \pm 0.2°C$, and atmospheric pressure. Examples of specific methods for measuring the expansion retention rate are described in detail in the Examples.

As a method of obtaining water-absorbing resin particles having an expansion retention rate of 98% or more, there is a method of increasing the uniformity of the hardness of the water-absorbing resin particles. For example, water-absorbing resin particles containing a uniform crosslinked structure tend to have a high degree of hardness uniformity.

The expansion retention rate of the water-absorbing resin particles may be 110% or less, or 108% or less, from the viewpoint of further improving the absorption amount of the absorber under pressure. From the same viewpoint, the volume $V_0$ of the swollen gel with pure water may be 20 to 25 mL.

The value of the non-pressurization DW after 10 minutes of the water-absorbing resin particles may be 40 mL/g or more, 45 mL/g or more, 50 mL/g or more, 55 mL/g or more, or 60 mL/g or more. Here, the non-pressurization DW is a water absorption rate represented by an amount of physiological saline absorbed by the water-absorbing resin particles until a predetermined time elapses after the water-absorbing resin particles come into contact with physiological saline (saline having a concentration of 0.9% by mass) under no pressure. The non-pressurization DW is represented by an absorption amount (mL) per 1 g of the water-absorbing resin particles before absorption of physiological saline. The value of the non-pressurization DW after 10 minutes means an absorption amount 10 minutes after the water-absorbing resin particles come into contact with physiological saline. When the value of the non-pressurization DW after 10 minutes of the water-absorbing resin particles is 40 mL/g or more, the absorption amount of the absorber under pressure can be further increased. The value of the non-pressurization DW after 10 minutes may be, for example, 80 mL/g or less, 75 mL/g or less, or 70 mL/g or less. A specific method for measuring the non-pressurization DW is described in detail in Examples.

The amount of water absorption of the water-absorbing resin particles with respect to physiological saline under pressure with a load of 4.14 kPa (hereinafter, sometimes referred to as "water absorption capacity under load") may be, for example, 8 mL/g or more, 12 mL/g or more, 15 mL/g or more, 18 mL/g or more, or 20 mL/g or more, and may be 35 mL/g or less, or 30 mL/g or less. The water absorption capacity under load is measured by the method described in Examples described later.

The water retention capacity of the water-absorbing resin particles with respect to physiological saline may be 30 g/g or more, 32 g/g or more, 35 g/g or more, 38 g/g or more, 39 g/g or more, 40 g/g or more, 41 g/g or more, or 42 g/g or more. The water-absorbing resin particles may have a water retention capacity of physiological saline of 60 g/g or less, 57 g/g or less, 55 g/g or less, 53 g/g or less, 50 g/g or less, or 48 g/g or less. The water-absorbing resin particles may have a water retention capacity in physiological saline of 30 to 60 g/g, 35 to 55 g/g, 38 to 53 g/g, 40 to 50 g/g, or 42 to 48 g/g. When the water retention capacity is within these ranges, the permeation rate of the absorber tends to increase, and liquid leakage from the absorber tends to be suppressed. The water retention capacity of the water-absorbing resin particles with respect to physiological saline is measured by the method described in Examples described later.

Examples of the shape of the water-absorbing resin particles according to the present embodiment include a substantially spherical shape, a crushed shape, and a granular shape. The median particle diameter of the water-absorbing resin particles according to the present embodiment may be 250 $\mu$m or more and 850 $\mu$m or less, 700 $\mu$m or less, or 600 $\mu$m or less, and may be 300 $\mu$m or more and 850 $\mu$m or less, 700 $\mu$m or less, or 600 $\mu$m or less. The water-absorbing resin particles according to the present embodiment may have a desired particle size distribution at the time of being obtained by a production method described later, but the particle size distribution may be adjusted by performing an operation such as particle size adjustment using classification with a sieve.

The water-absorbing resin particles according to the present embodiment may include, for example, a crosslinked polymer formed by polymerization of a monomer including an ethylenically unsaturated monomer. The crosslinked polymer has a monomer unit derived from an ethylenically unsaturated monomer.

The water-absorbing resin particles can be produced by a method including a step of polymerizing a monomer containing an ethylenically unsaturated monomer. Examples of the polymerization method include a reverse phase suspension polymerization method, an aqueous solution polymerization method, a bulk polymerization method, and a precipitation polymerization method. The polymerization method may be a reverse phase suspension polymerization method or an aqueous solution polymerization method from the viewpoint of ensuring good water absorption characteristics of the obtained water absorbing resin particles and easily controlling the polymerization reaction. In the following, a reverse phase suspension polymerization method will be described as an example of a method for polymerizing an ethylenically unsaturated monomer.

The ethylenically unsaturated monomer may be water-soluble. Examples of the water-soluble ethylenically unsaturated monomer include (meth) acrylic acid and salts thereof, 2-(meth)acrylamide-2-methylpropanesulfonic acid and salts thereof, (meth)acrylamide, N,N-dimethyl(meth)acrylamide, 2-hydroxyethyl(meth)acrylate, N-methylol(meth)acrylamide, polyethylene glycol mono(meth)acrylate, N,N-diethylaminoethyl(meth)acrylate, N,N-diethylaminopropyl(meth)acrylate, and diethylaminopropyl(meth)acrylamide. In the case where the ethylenically unsaturated monomer has an amino group, the amino group may be quaternized. The ethylenically unsaturated monomer may be used alone or in combination of two or more. The functional group such as a carboxyl group or an amino group of the monomer can function as a crosslinkable functional group in the surface crosslinking step described later.

From the viewpoint of industrial availability, the ethylenically unsaturated monomer may include at least one kind of compound selected from the group consisting of (meth)acrylic acid and salts thereof, acrylamide, methacrylamide, and N,N-dimethylacrylamide, or may include at least one kind of compound selected from the group consisting of (meth)acrylic acid and salts thereof, and acrylamide. From the viewpoint of further enhancing the water absorption properties, the ethylenically unsaturated monomer may contain at least one kind of compound selected from the group consisting of (meth)acrylic acid and salts thereof.

The ethylenically unsaturated monomer is usually preferably used as an aqueous solution. The concentration of the ethylenically unsaturated monomer in the aqueous solution containing the ethylenically unsaturated monomer (hereinafter, simply referred to as "aqueous monomer solution") may be 20% by mass or more and a saturated concentration or less, 25 to 70% by mass, or 30 to 55% by mass. Examples of water used in the aqueous solution include tap water, distilled water, and ion-exchanged water.

As the monomer for obtaining the water-absorbing resin particles, a monomer other than the above-mentioned ethylenically unsaturated monomer may be used. Such monomers can be used, for example, by mixing with an aqueous solution containing the above-mentioned ethylenically unsaturated monomer. The amount of the ethylenically unsaturated monomer used may be 70 to 100 mol% relative to the total amount of the monomers. The ratio of (meth)acrylic acid and a salt thereof may be 70 to 100 mol% with respect to the total amount of monomers.

When the ethylenically unsaturated monomer has an acid group, the aqueous monomer solution may be used after neutralizing the acid group with an alkaline neutralizing agent. The degree of neutralization of the ethylenically unsaturated monomer with the alkaline neutralizing agent may be 10 to 100 mol%, 50 to 90 mol%, or 60 to 80 mol% of the acidic groups in the ethylenically unsaturated monomer from the viewpoint of increasing the osmotic pressure of the resulting water-absorbing resin particles and further enhancing the water-absorbing properties (water absorption capacity and the like). Examples of the alkaline neutralizing agent include alkali metal salts such as sodium hydroxide, sodium carbonate, sodium hydrogen carbonate, potassium hydroxide and potassium carbonate; and ammonia. The alkaline neutralizer may be used alone or in combination of two or more. The alkaline neutralizing agent may be used in the form of an aqueous solution in order to simplify the neutralization operation. The neutralization of the acid group of the ethylenically unsaturated monomer can be carried out, for example, by adding dropwise an aqueous solution of sodium hydroxide, potassium hydroxide or the like to the aqueous monomer solution and mixing them.

In the reverse phase suspension polymerization method, an aqueous monomer solution is dispersed in a hydrocarbon dispersion medium in the presence of a surfactant, and an ethylenically unsaturated monomer can be polymerized using a radical polymerization initiator or the like. As the radical polymerization initiator, a water-soluble radical polymerization initiator can be used.

Examples of the surfactant include nonionic surfactants and anionic surfactants. Examples of the nonionic surfactant include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol-fatty acid esters, polyoxyethylene alkyl ethers, polyoxyethylene alkylphenyl ethers, polyoxyethylene castor oils, polyoxyethylene hydrogenated castor oils, alkylaryl formaldehyde-condensed polyoxyethylene ethers, polyoxyethylene polyoxypropylene block copolymers, polyoxyethylene polyoxypropylene alkyl ethers, and polyethylene glycol fatty acid esters. Examples of the anionic surfactant include fatty acid salts, alkylbenzene sulfonates, alkyl methyl taurates, polyoxyethylene alkyl phenyl ether sulfates, polyoxyethylene alkyl ether sulfonates, polyoxyethylene alkyl ether phosphates, and polyoxyethylene alkyl allyl ether phosphates. The surfactant may be used alone or in combination of two or more thereof.

The surfactant may contain at least one kind of compound selected from the group consisting of a sorbitan fatty acid ester, a polyglycerol fatty acid ester, and a sucrose fatty acid ester, from the viewpoint that a W/O type reverse phase suspension state is good, water-absorbing resin particles having a suitable particle diameter are easily obtained, and industrially easily available. The surfactant may contain a sucrose fatty acid ester or a sucrose stearic acid ester from the viewpoint of easily improving the water absorption characteristics of the obtained water absorbing resin particles.

The used amount of the surfactant may be 0.05 to 10 parts by mass, 0.08 to 5 parts by mass, or 0.1 to 3 parts by mass with respect to 100 parts by mass of the aqueous monomer solution from the viewpoint of sufficiently obtaining the effect on the used amount and from the viewpoint of economy.

In the reverse phase suspension polymerization, a polymeric dispersant may be used in combination with the above-described surfactant. Examples of the polymeric dispersant include maleic anhydride-modified polyethylene, maleic anhydride-modified polypropylene, maleic anhydride-modified ethylene-propylene copolymer, maleic anhydride-modified EPDM (ethylene-propylene-diene terpolymer), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethylcellulose, and ethylhydroxyethyl cellulose. The polymeric dispersant may be used alone or in combination of two or more. From the viewpoint of better monomer dispersion stability, the polymeric dispersant may contain at least one selected from the group consisting of maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer.

The amount of the polymeric dispersant used may be 0.05 to 10 parts by mass, 0.08 to 5 parts by mass, or 0.1 to 3 parts by mass with respect to 100 parts by mass of the aqueous monomer solution from the viewpoint of sufficiently obtaining the effect on the amount used and from the viewpoint of economy.

The hydrocarbon dispersion medium may contain at least one kind of compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-imethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone or in combination of two or more.

From the viewpoint of industrial availability and stable quality, the hydrocarbon dispersion medium may contain at least one selected from the group consisting of n-heptane and cyclohexane. From the same viewpoint, as the mixture of the hydrocarbon dispersion media, for example, commercially available Exxsol heptane (manufactured by Exxon Mobil Corporation: containing n-heptane and 75 to 85% of isomeric hydrocarbons) may be used.

The amount of the hydrocarbon dispersion medium used may be 30 to 1000 parts by mass, 40 to 500 parts by

EP 3 936 539 A1

mass, or 50 to 300 parts by mass with respect to 100 parts by mass of the aqueous monomer solution from the viewpoint of appropriately removing the polymerization heat and easily controlling the polymerization temperature. When the amount of the hydrocarbon dispersion medium used is 30 parts by mass or more, the polymerization temperature tends to be easily controlled. When the amount of the hydrocarbon dispersion medium used is 1000 parts by mass or less, the productivity of polymerization tends to be improved, which is economical.

The radical polymerization initiator may be water-soluble. Examples of the water-soluble radical polymerization initiator include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butylperoxyacetate, t-butylperoxyisobutyrate, t-butylperoxypivalate, and hydrogen peroxide; 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazoline-2-yl)propane] dihydrochloride, 2,2'-azobis{2-[1-(2-hydroxyethyl)-2-imidazolin-4-yl]propane} dihydrochloride, 2,2'-azobis{2-methyl-N-[1,1-bis (hydroxymethyl)-2-hydroxyethyl]propionamide}, 2,2'-azo-bis[2-methyl-N-(2-hydroxyethyl)-propionamide], and 4,4'-azobis(4-cyanovaleric acid). The radical polymerization initiator may be used alone or in combination of two or more. The radical polymerization initiator may be at least one selected from the group consisting of potassium persulfate, ammonium persulfate, sodium persulfate, 2,2'-azobis(2-amidinopropane) dihydrochloride, 2,2'-azobis[2-(2-imidazoline-2- yl) propane] dihydrochloride, and 2,2'-azobis{2-[1-(2-hydroxyethyl)-2-imidazoline-2-yl]propane} dihydrochloride.

The amount of the radical polymerization initiator used may be 0.00005 to 0.01 mol relative to 1 mol of the ethylenically unsaturated monomer. When the amount of the radical polymerization initiator used is 0.00005 mol or more, the polymerization reaction does not require a long time and is efficient. When the amount of the radical polymerization initiator used is 0.01 mol or less, the occurrence of a rapid polymerization reaction is easily suppressed.

The above-mentioned radical polymerization initiator can also be used as a redox polymerization initiator in combination with a reducing agent such as sodium sulfite, sodium hydrogensulfite, ferrous sulfate or L-ascorbic acid.

In the polymerization reaction, the aqueous monomer solution used for the polymerization may contain a chain transfer agent. Examples of the chain transfer agent include hypophosphites, thiols, thiol acids, second alcohols, and amines.

In order to control the particle size of the water-absorbing resin particles, the aqueous monomer solution used for polymerization may contain a thickener. Examples of the thickener include hydroxyethylcellulose, hydroxypropylcellulose, methylcellulose, carboxymethylcellulose, polyacrylic acid, polyethylene glycol, polyacrylamide, polyethyleneimine, dextrin, sodium alginate, polyvinylalcohol, polyvinylpyrrolidone, and polyethylene oxide. If the stirring speed during polymerization is the same, the higher viscosity of the aqueous monomer solution tends to result in the larger median particle diameter of the obtained particles.

Crosslinking by self-crosslinking may occur during polymerization, and crosslinking may be performed by using an internal crosslinking agent. When the internal crosslinking agent is used, it is easy to control the water absorption characteristics of the water-absorbing resin particles. The internal crosslinking agent is typically added to the reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol and polyglycerin; unsaturated polyesters obtained by reacting the above-mentioned polyols with unsaturated acids (maleic acid, fumaric acid, etc.); bis(meth)acrylamides such as N,N'-methylenebis(meth)acrylamide; di or tri (meth)acrylic acid esters obtained by reacting polyepoxide with (meth) acrylic acid; carbamylesters obtained by reacting polyisocyanate (tolylenediisocyanate, hexamethylenediisocyanate, etc.) with hydroxyethyl(meth)acrylate; compounds having two or more polymerizable unsaturated groups such as allylated starch, allylated cellulose, diallyl phthalate, N,N',N''-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methylepichlorohydrin; and compounds having two or more reactive functional groups such as isocyanate compounds (2,4-tolylene diisocyanate, and hexamethylene diisocyanate). The internal crosslinking agent may be used alone or in combination of two or more thereof. From the viewpoint of easily enhancing the uniformity of crosslinking in the water-absorbing resin particles, the internal crosslinking agent may include a polyglycidyl compound or a diglycidyl ether compound, and may include at least one selected from the group consisting of (poly)ethylene glycol diglycidyl ether, (poly)propylene glycol diglycidyl ether, and (poly)glycerin diglycidyl ether.

The amount of the internal crosslinking agent to be used may be 0 mmol or more, 0.02 mmol or more, 0.03 mmol or more, 0.04 mmol or more, or 0.05 mmol or more, and may be 0.1 mol or less per 1 mol of the ethylenically unsaturated monomer from the viewpoint that the water-soluble property is suppressed by appropriately crosslinking the obtained polymer and a sufficient water absorption capacity is easily obtained.

The reverse phase suspension polymerization can be carried out in an oil-in-water system by heating a mixture of an aqueous phase containing an ethylenically unsaturated monomer, a radical polymerization initiator and, if necessary, an internal crosslinking agent and an oil phase containing a hydrocarbon dispersant and, if necessary, a surfactant and

6

a polymeric dispersant under stirring.

When the reverse phase suspension polymerization is performed, an aqueous monomer solution containing an ethylenically unsaturated monomer is dispersed in a hydrocarbon dispersion medium in the presence of a surfactant (and, if necessary, a polymeric dispersant). At this time, the surfactant, the polymeric dispersant, and the like may be added either before or after the addition of the aqueous monomer solution as long as they are added before the start of the polymerization reaction.

From the viewpoint of easily reducing the amount of the hydrocarbon dispersion medium remaining in the resulting water-absorbing resin, the polymerization may be carried out after dispersing the aqueous monomer solution in the hydrocarbon dispersion medium in which the polymeric dispersant is dispersed and then further dispersing the surfactant.

The reverse phase suspension polymerization can be carried out in one stage or in multiple stages of two or more stages. The reverse phase suspension polymerization may be performed in 2 to 3 stages from the viewpoint of enhancing productivity.

When the reverse phase suspension polymerization is carried out in two or more stages, after the reverse phase suspension polymerization in the first stage is carried out, the ethylenically unsaturated monomer is added to and mixed with the reaction mixture obtained in the polymerization reaction in the first stage, and the reverse phase suspension polymerization in the second and subsequent stages may be carried out in the same manner as in the first stage. In the reverse phase suspension polymerization in each of the second and subsequent stages, the above-mentioned radical polymerization initiator may be added in addition to the ethylenically unsaturated monomer within the range of the molar ratio of each component to the ethylenically unsaturated monomer based on the amount of the ethylenically unsaturated monomer added in the reverse phase suspension polymerization in each of the second and subsequent stages. In the reverse phase suspension polymerization in each of the second and subsequent stages, an internal crosslinking agent may be used as necessary. When an internal crosslinking agent is used, the reverse phase suspension polymerization may be carried out by adding the internal crosslinking agent within the above-described molar ratio of each component to the ethylenically unsaturated monomer based on the amount of the ethylenically unsaturated monomer supplied to each stage.

The temperature of the polymerization reaction varies depending on the radical polymerization initiator used. The temperature of the polymerization reaction may be from 20 to 150°C, or from 40 to 120°C, from the viewpoint of enhancing economic efficiency by rapidly proceeding the polymerization and shortening the polymerization time, and easily removing the polymerization heat to perform the reaction smoothly. The reaction time is usually 0.5 to 4 hours. The completion of the polymerization reaction can be confirmed by, for example, stopping the temperature rise in the reaction system. Thus, the polymer of the ethylenically unsaturated monomer is usually obtained in the form of a hydrogel-like polymer.

After the polymerization, a crosslinking agent may be added to the obtained hydrogel-like polymer, followed by heating to perform post-polymerization crosslinking. By carrying out post-polymerization crosslinking, the degree of crosslinking of the hydrogel-like polymer can be increased, thereby further improving the water absorption properties of the water-absorbing resin particles.

Examples of the crosslinking agent include polyols such as ethylene glycol, propylene glycol, 1,4-butanediol, trimethylolpropane, glycerin, polyoxyethylene glycol, polyoxypropylene glycol and polyglycerin; compounds having two or more epoxy groups such as (poly)ethylene glycol diglycidyl ether, (poly)propylene glycol diglycidyl ether and (poly)glycerin diglycidyl ether ; haloepoxy compounds such as epichlorohydrin, epibromhydrin and $\alpha$-methylepichlorohydrin; compounds having two or more isocyanate groups such as 2,4-tolylene diisocyanate and hexamethylene diisocyanate; oxazoline compounds such as 1,2-ethylene bisoxazoline; carbonate compounds such as ethylene carbonate; and alkyladipamide compounds such as bis [N,N-di($\beta$-hydroxyethyl)]adipamide. Crosslinking agents for post-polymerization crosslinking may include polyglycidyl compounds such as (poly)ethylene glycol diglycidyl ether, (poly)glycerin diglycidyl ether, (poly)glycerin triglycidyl ether, (poly)propylene glycol polyglycidyl ether, polyglycerol polyglycidyl ether, and the like. These crosslinking agents may be used alone or in combination of two or more.

The amount of the crosslinking agent used in the post-polymerization crosslinking may be 0 to 0.03 mol, 0 to 0.01 mol, or 0.00001 to 0.005 mol per 1 mol of the ethylenically unsaturated monomer, from the viewpoint of appropriately crosslinking the obtained hydrogel-like polymer to exhibit suitable water absorption characteristics.

The crosslinking agent for post-polymerization crosslinking may be added after polymerization of the ethylenically unsaturated monomer used for polymerization. In the case of multistage polymerization, a crosslinking agent may be added after the multistage polymerization. The crosslinking agent for post-polymerization crosslinking may be added in the range of [water content immediately after polymerization ± 3% by mass] from the viewpoint of water content (described later) in consideration of heat generation during and after polymerization, retention due to process delay, opening of the system during addition of the crosslinking agent, and variation in water content due to addition of water accompanying addition of the crosslinking agent.

Subsequently, the obtained hydrogel-like polymer is dried to remove water. By the drying, polymer particles containing the polymer of the ethylenically unsaturated monomer are obtained. Examples of the drying method include (a) a method in which the hydrogel-like polymer dispersed in the hydrocarbon dispersion medium is heated from the outside to carry out azeotropic distillation and the hydrocarbon dispersion medium is refluxed to remove water, (b) a method in which the hydrogel-like polymer is taken out by decantation and dried under reduced pressure, and (c) a method in which the hydrogel-like polymer is separated by filtration and dried under reduced pressure. The method (a) may be used for simplicity in the production process.

The particle size of the water-absorbing resin particles can be adjusted by adjusting the rotation speed of the stirring blades of the stirrer during the polymerization reaction, or by adding a flocculant to the system after the polymerization reaction or at the initial stage of drying. By adding the flocculant, the particle diameter of the obtained water-absorbing resin particles can be increased. As the flocculant, an inorganic flocculant can be used. Examples of the inorganic flocculant (for example, a powdery inorganic flocculant) include silica, zeolite, bentonite, aluminum oxide, talc, titanium dioxide, kaolin, clay, and hydrotalcite. From the viewpoint of better flocculation effect, the flocculant may contain at least one selected from the group consisting of silica, aluminum oxide, talc, and kaolin. By controlling the rotation speed of the stirring blade within an appropriate range, the uniformity of crosslinking in the water-absorbing resin particles is easily improved.

In the reverse phase suspension polymerization, the flocculant may be added by a method in which the flocculant is dispersed in advance in a hydrocarbon dispersion medium of the same type as that used in the polymerization or in water and then mixed into the hydrocarbon dispersion medium containing the hydrogel-like polymer under stirring.

The addition amount of the flocculant may be 0.001 to 1 parts by mass, 0.005 to 0.5 parts by mass, or 0.01 to 0.2 parts by mass with respect to 100 parts by mass of the ethylenically unsaturated monomer used for polymerization. When the addition amount of the flocculant is within the above range, water-absorbing resin particles having a target particle size distribution are easily obtained.

The polymerization reaction can be carried out using various stirrers having stirring blades. As the stirring blade, a flat blade, a lattice blade, a paddle blade, a propeller blade, an anchor blade, a turbine blade, a pfaudler blade, a ribbon blade, a full-zone blade, a maxblend blade, or the like can be used. The flat blade includes a shaft (stirring shaft) and a flat plate portion (stirring portion) disposed around the shaft. Further, the flat plate portion may have a slit or the like. When a flat blade is used as the stirring blade, the uniformity of crosslinking of the polymer in the formed polymer particles tends to be high. When the uniformity of crosslinking is high, the uniformity of the hardness of the water-absorbing resin particles becomes high. The water-absorbing resin particles having high hardness uniformity tend to exhibit an expansion retention rate of 98% or more.

In the production of the water-absorbing resin particles, the surface portion of the hydrogel-like polymer may be crosslinked (surface crosslinked) using a crosslinking agent in the drying step or in any subsequent step. By performing surface crosslinking, the water absorption characteristics of the water absorbing resin particles can be easily controlled. The surface crosslinking may be performed at a timing when the hydrogel-like polymer has a specific water content. The surface crosslinking may be performed at the time when the water content of the hydrogel-like polymer is 5 to 50 mass%, 10 to 40 mass%, or 15 to 35 mass%. The water content (% by mass) of the hydrogel-like polymer is calculated by the following formula.

$$\text{Water content} = [\text{Ww} / (\text{Ww} + \text{Ws})] \times 100$$

Ww: The water content of the water-containing gel-like polymer obtained by subtracting the water content discharged to the outside of the system in the drying step from the water content contained in the aqueous monomer solution before polymerization in the entire polymerization step, and adding the water content used as necessary when mixing the flocculant, the surface crosslinking agent, and the like.

Ws is a solid content calculated from the charged amounts of materials such as an ethylenically unsaturated monomer constituting the hydrogel-like polymer, a crosslinking agent and an initiator.

Examples of the crosslinking agent for surface crosslinking (surface crosslinking agent) include compounds having two or more reactive functional groups. Examples of the crosslinking agent include polyols such as ethylene glycol, propylene glycol, 1,4-butanediol, trimethylolpropane, glycerin, polyoxyethylene glycol, polyoxypropylene glycol and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether (poly)propylene glycol polyglycidyl ether and (poly)glycerol polyglycidyl ether; haloepoxy compounds such as epichlorohydrin, epibromhydrin and $\alpha$-methylepichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane ethanol, 3-ethyl-3-oxetane oxetane ethanol, 3-butyl-3-oxetane ethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. The crosslinking agent may be used alone or in combination of two or more thereof. The crosslinking agent may include a polyglycidyl compound, and may include at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The amount of the surface crosslinking agent to be used may be usually 0.00001 to 0.02 mol, 0.00005 to 0.01 mol, or 0.0001 to 0.005 mol relative to 1 mol of the ethylenically unsaturated monomer to be used for polymerization, from the viewpoint of appropriately crosslinking the obtained hydrogel-like polymer to exhibit suitable water absorption characteristics. When the amount of the surface crosslinking agent used is 0.00001 mol or more, the crosslinking density in the surface portion of the water-absorbing resin particles is sufficiently increased, and the gel strength of the water-absorbing resin particles is easily increased. When the amount of the surface crosslinking agent used is 0.02 mol or less, the water absorption amount of the water-absorbing resin particles is easily increased.

After the surface crosslinking, the water and the hydrocarbon dispersion medium are distilled off by a known method to obtain polymer particles which are a dried product of the surface crosslinked hydrogel-like polymer.

The water-absorbing resin particles according to the present embodiment may be composed only of polymer particles, but may further contain various additional components selected from, for example, a gel stabilizer, a metal chelating agent, and a flowability improver (lubricant). The additional components can be disposed within the polymer particles, on the surface of the polymer particles, or both. The additional component may be a flow improver (lubricant). The flow improver may be an inorganic particle. Examples of the inorganic particle include a silica particle such as amorphous silica.

The water-absorbing resin particles may include a plurality of inorganic particles disposed on the surface of the polymer particles. For example, the inorganic particles can be disposed on the surface of the polymer particles by mixing the polymer particles and the inorganic particles. The inorganic particles may be silica particles such as amorphous silica. When the water-absorbing resin particles include inorganic particles disposed on the surfaces of the polymer particles, the ratio of the inorganic particles to the mass of the polymer particles may be 0.2% by mass or more, 0.5% by mass or more, 1.0% by mass or more, or 1.5% by mass or more, and may be 5.0% by mass or less, or 3.5% by mass or less. The inorganic particles herein usually have a small size compared to the size of the polymer particles. For example, the average particle diameter of the inorganic particles may be 0.1 to 50 $\mu$m, 0.5 to 30 $\mu$m, or 1 to 20 $\mu$m. The average particle diameter herein can be a value measured by a dynamic light scattering method or a laser diffraction / scattering method.

The absorber according to an embodiment contains the water-absorbing resin particles according to the present embodiment. The absorber according to the present embodiment can contain a fibrous material, and is, for example, a mixture containing water-absorbing resin particles and a fibrous material. The configuration of the absorber may be, for example, a configuration in which the water-absorbing resin particles and the fibrous material are uniformly mixed, a configuration in which the water-absorbing resin particles are sandwiched between fibrous materials formed in a sheet shape or a layer shape, or another configuration.

Examples of the fibrous material include finely pulverized wood pulp; cotton; cotton linter; rayon; cellulosic fibers such as cellulose acetate; synthetic fibers such as polyamide, polyester, and polyolefin; and mixtures of these fibers. The fibrous material may be used alone or in combination of two or more. As the fibrous material, hydrophilic fibers can be used.

The mass ratio of the water-absorbing resin particles in the absorber may be 2 to 100 mass%, 10 to 80 mass%, or 20 to 60 mass% with respect to the total of the water-absorbing resin particles and the fibrous material.

In order to enhance the shape retention before and during the use of the absorber, the fibers may be bonded to each other by adding an adhesive binder to the fibrous material. Examples of the adhesive binder include thermal bonding synthetic fibers, hot melt adhesives, and adhesive emulsions. The adhesive binder may be used alone or in combination of two or more.

Examples of the thermal bonding synthetic fiber include a total fusion type binder such as polyethylene, polypropylene, and ethylene-propylene copolymer; and a non-total fusion type binder having a side-by-side or core-in-sheath structure of polypropylene and polyethylene. In the non-total fusion type binder described above, only the polyethylene portion can be thermal-bonded.

Examples of the hot melt adhesive include a mixture of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and amorphous polypropylene, and a tackifier, a plasticizer, an antioxidant, and the like.

Examples of the adhesive emulsion include a polymer of at least one monomer selected from the group consisting of methyl methacrylate, styrene, acrylonitrile, 2-ethylhexylacrylate, butylacrylate, butadiene, ethylene, and vinyl acetate.

The absorber according to the present embodiment may contain an inorganic powder (for example, amorphous silica), a deodorant, an antibacterial agent, a perfume, and the like. When the water-absorbing resin particles contain inorganic particles, the absorber may contain an inorganic powder separately from the inorganic particles in the water-absorbing resin particles.

The shape of the absorber according to the present embodiment is not particularly limited, and may be, for example, a sheet shape. The thickness of the absorber (for example, the thickness of the sheet-shaped absorber) may be, for example, 0.1 to 20 mm or 0.3 to 15 mm.

The absorbent article according to the present embodiment includes the absorber according to the present embodiment. Examples of the absorbent article according to the present embodiment include a core wrap that retains the shape of the absorber; a liquid permeable sheet disposed at the outermost portion on the side where the liquid to be absorbed enters; and a liquid impermeable sheet disposed at the outermost portion on the side opposite to the side where the liquid to be absorbed enters. Examples of the absorbent article include diapers (for example, disposable diapers), training pants for toilets, incontinence pads, sanitary materials (sanitary napkins, tampons, and the like), perspiration pads, pet sheets, members for simple toilets, animal excrement disposal materials, and the like.

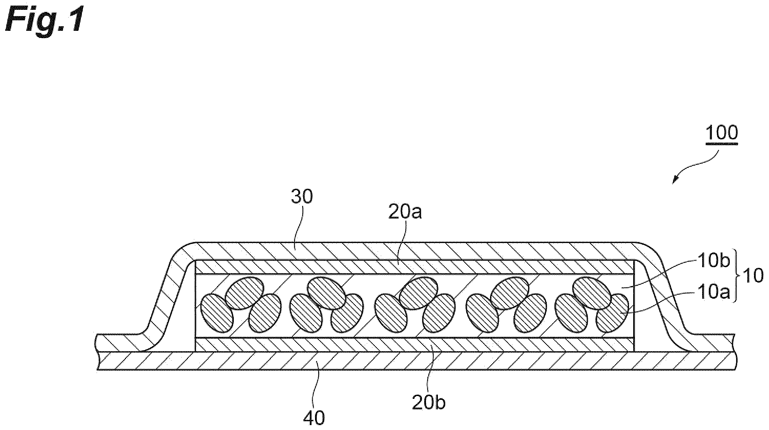

FIG. 1 is a cross-sectional view illustrating an example of an absorbent article. An absorbent article 100 shown in FIG. 1 includes an absorber 10, core wraps 20a, 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In FIG. 1, some parts are illustrated as having a gap between the members, but the members may be in close contact with each other without the gap.

The absorber 10 includes water-absorbing resin particles 10a according to the present embodiment and a fiber layer 10b containing a fibrous material. The water-absorbing resin particles 10a are dispersed in the fiber layer 10b.

The core wrap 20a is disposed on one surface side of the absorber 10 (upper side of the absorber 10 in FIG. 1) in a state of being in contact with the absorber 10. The core wrap 20b is disposed on the other surface side of the absorber 10 (the lower side of the absorber 10 in FIG. 1) in a state of being in contact with the absorber 10. The absorber 10 is disposed between the core wrap 20a and the core wrap 20b. Examples of the core wraps 20a, 20b include tissues and nonwoven fabrics. The core wrap 20a and the core wrap 20b have, for example, main surfaces of the same size as the absorber 10.

The liquid-permeable sheet 30 is disposed at the outermost portion on the side where the liquid to be absorbed enters. The liquid permeable sheet 30 is disposed on the core wrap 20a in a state of being in contact with the core wrap 20a. The liquid impermeable sheet 40 is disposed at the outermost portion of the absorbent article 100 on the side opposite to the liquid permeable sheet 30. The liquid impermeable sheet 40 is disposed on the lower side of the core wrap 20b in a state of being in contact with the core wrap 20b. The liquid permeable sheet 30 and the liquid impermeable sheet 40 have, for example, main surfaces wider than the main surface of the absorber 10, and the outer edge portions of the liquid permeable sheet 30 and the liquid impermeable sheet 40 extend around the absorber 10 and the core wraps 20a, 20b.

The size relationship among the absorber 10, the core wraps 20a, 20b, the liquid permeable sheet 30, and the liquid impermeable sheet 40 is not particularly limited, and is appropriately adjusted according to the use of the absorbent article or the like. The absorber 10 shown in FIG. 1 is held in shape by being sandwiched between 2 core wraps 20a, 20b. The method of retaining the shape by the core wrap for retaining the shape of the absorber is not limited thereto, and for example, the absorber may be sandwiched between one folded core wrap. The core wrap may form a bag body, and the absorber may be disposed therein.

The liquid permeable sheet 30 may be a sheet formed from a resin or fiber commonly used in the art. From the viewpoint of liquid permeability, flexibility, and strength when used in an absorbent article, the liquid permeable sheet 30 may contain, for example, polyolefins such as polyethylene (PE) and polypropylene (PP), polyesters such as polyethylene terephthalate (PET), polytrimethylene terephthalate (PTT), and polyethylene naphthalate (PEN), polyamides such as nylon, and synthetic resins such as rayon, or natural fibers including cotton, silk, hemp, or pulp (cellulose). From the viewpoint of increasing the strength of the liquid permeable sheet 30, the liquid permeable sheet 30 may contain synthetic fibers. The synthetic fibers may in particular be polyolefin fibers, polyester fibers or combinations thereof. These materials may be used alone or in combination of two or more.

The liquid permeable sheet 30 may be a nonwoven fabric, a porous sheet, or a combination thereof. The nonwoven fabric is a sheet in which fibers are intertwined without being woven. The nonwoven fabric may be a nonwoven fabric composed of short fibers (that is, staple) (short fiber nonwoven fabric) or a nonwoven fabric composed of long fibers (that is, filament) (long fiber nonwoven fabric). The staple may generally have a fiber length of several 100 mm or less, but is not limited thereto.

The liquid permeable sheet 30 may be a thermally bonded nonwoven fabric, an air-through nonwoven fabric, a resin-bonded nonwoven fabric, a spunbonded nonwoven fabric, a melt-blown nonwoven fabric, an air-laid nonwoven fabric, a spunlace nonwoven fabric, a point-bonded nonwoven fabric, or a stack of two or more kinds of nonwoven fabrics selected from these. These nonwoven fabrics can be formed of, for example, the above-mentioned synthetic fibers or natural fibers. The stack of the two or more types of nonwoven fabrics may be, for example, a spunbond / meltblown / spunbond nonwoven fabric which is a composite nonwoven fabric having a spunbond nonwoven fabric, a meltblown nonwoven fabric and a spunbond nonwoven fabric laminated in this order. From the viewpoint of suppressing liquid leakage, a thermal bond nonwoven fabric, an air-through nonwoven fabric, a spunbond nonwoven fabric, or a spunbond / meltblown / spunbond nonwoven fabric may be used. In the spunbonded / meltblown / spunbonded nonwoven fabric, two or more layers of meltblown nonwoven fabric may be provided between two layers of spunbonded nonwoven fabric.

The nonwoven fabric used as the liquid permeable sheet 30 may have appropriate hydrophilicity from the viewpoint of the liquid absorption performance of the absorbent article. From this viewpoint, the liquid permeable sheet 30 may be a nonwoven fabric having a hydrophilicity of 5 to 200 as measured according to the measurement method of Paper and Pulp Test Method No. 68 (2000) by the Technical Association of the Pulp and Paper Industry. The hydrophilicity of the nonwoven fabric may be 10 to 150. For details of the paper pulp test method No. 68, reference can be made, for example, to WO 20/11/086843.

The nonwoven fabric having hydrophilicity as described above may be formed of fibers exhibiting appropriate hydrophilicity such as rayon fibers, or may be formed of fibers obtained by subjecting hydrophobic chemical fibers such as polyolefin fibers and polyester fibers to a hydrophilic treatment. Examples of a method for obtaining a nonwoven fabric containing a hydrophobic chemical fiber subjected to a hydrophilization treatment include a method for obtaining a nonwoven fabric by a spunbonding method using a mixture of a hydrophobic chemical fiber and a hydrophilizing agent, a method for producing a spunbonded nonwoven fabric using a hydrophobic chemical fiber with a hydrophilizing agent, and a method for impregnating a spunbonded nonwoven fabric obtained using a hydrophobic chemical fiber with a hydrophilizing agent. Examples of the hydrophilizing agent include anionic surfactants such as aliphatic sulfonates and higher alcohol sulfates, cationic surfactants such as fourth ammonium salts, nonionic surfactants such as polyethylene glycol fatty acid esters, polyglycerin fatty acid esters, and sorbitan fatty acid esters, silicone-based surfactants such as polyoxyalkylene-modified silicones, and stain-release agents composed of polyester, polyamide, acrylic, and urethane resins.

The liquid permeable sheet 30 may be a nonwoven fabric that is moderately bulky and has a large basis weight from the viewpoint of being able to impart good liquid permeability, flexibility, strength, and cushioning properties to the absorbent article and from the viewpoint of increasing the liquid permeation rate of the absorbent article. The basis weight of the nonwoven fabric used for the liquid permeable sheet 30 may be 5 to 200 $g/m^2$, 8 to 150 $g/m^2$, or 10 to 100 $g/m^2$. The thickness of the nonwoven fabric used for the liquid permeable sheet 30 may be 20 to 1400 $\mu$m, 50 to 1200 $\mu$m, or 80 to 1000 $\mu$m.

The liquid impermeable sheet 40 is disposed at the outermost portion of the absorbent article 100 on the side opposite to the liquid permeable sheet 30. The liquid impermeable sheet 40 is disposed on the lower side of the core wrap 20b in a state of being in contact with the core wrap 20b. The liquid impermeable sheet 40 has, for example, a main surface wider than the main surface of the absorber 10, and the outer edge portion of the liquid impermeable sheet 40 extends around the absorber 10 and the core wraps 20a, 20b. The liquid-impermeable sheet 40 prevents the liquid absorbed by the absorber 10 from leaking out from the liquid-impermeable sheet 40 side.

Examples of the liquid-impermeable sheet 40 include a sheet made of a synthetic resin such as polyethylene, polypropylene, or polyvinyl chloride, a sheet made of a nonwoven fabric such as a spunbond / meltblown / spunbond (SMS) nonwoven fabric in which a water-resistant meltblown nonwoven fabric is sandwiched between high-strength spunbond nonwoven fabrics, and a sheet made of a composite material of these synthetic resins and a nonwoven fabric (for example, a spunbond nonwoven fabric or a spunlace nonwoven fabric). The liquid-impermeable sheet 40 may have air permeability from the viewpoint of reducing stuffiness during wearing and reducing discomfort given to the wearer. As the liquid impermeable sheet 40, a sheet made of a synthetic resin mainly composed of a low density polyethylene (LDPE) resin can be used. From the viewpoint of securing flexibility so as not to impair the wearing feeling of the absorbent article, the liquid impermeable sheet 40 may be a sheet made of a synthetic resin having a basis weight of 10 to 50 $g/m^2$, for example.

The absorbent article 100 can be produced, for example, by a method including disposing the absorber 10 between the core wraps 20a, 20b and disposing them between the liquid permeable sheet 30 and the liquid impermeable sheet 40. The stack in which the liquid impermeable sheet 40, the core wrap 20b, the absorber 10, the core wrap 20a, and the liquid permeable sheet 30 are laminated in this order is pressurized as necessary.

The absorber 10 is formed by mixing water-absorbing resin particles 10a with a fibrous material. The absorbing resin particles exhibiting an expansion retention rate of 98% or more may be selected and selectively used to form the absorber 10.

The expansion retention rate of the water-absorbing resin particles 10a can be used as an index for increasing the absorption amount of the absorber 10 containing the water-absorbing resin particles 10a under pressure. The absorption amount of the absorber 10 under pressure is increased by a method including increasing the swelling ratio of the water-absorbing resin particles 10a. For example, the water-absorbing resin particles 10a having an expansion retention rate of a predetermined value (for example, 98%) or more may be selected. Alternatively, the expansion retention rate of the water-absorbing resin particles can also be set to a predetermined value (for example, 98%) or more by selecting the production conditions of the water-absorbing resin particles so as to increase the uniformity of crosslinking in the water-absorbing resin particles. Examples of the method for enhancing the uniformity of crosslinking in the water-absorbing resin particles include the use of a flat blade as a stirring blade at the time of polymerization reaction, the selection of rotation speed of stirring in an appropriate range, the employment of conditions under which heat can be easily supplied and removed (for example, reaction in a hydrocarbon dispersion medium), and the use of a crosslinking agent having suitably high reactivity.

According to the present embodiment, a liquid absorbing method using the water-absorbing resin particles, the absorber, or the absorbent article according to the present embodiment can be provided. The liquid absorbing method according to the present embodiment includes a step of bringing a liquid to be absorbed into contact with the water-absorbing resin particles, the absorber, or the absorbent article according to the present embodiment.

EXAMPLES

Hereinafter, the present invention will be described in more detail with reference to examples. However, the present invention is not limited to these examples.

1. Production of Water-Absorbing Resin Particles

Example 1

First stage polymerization reaction

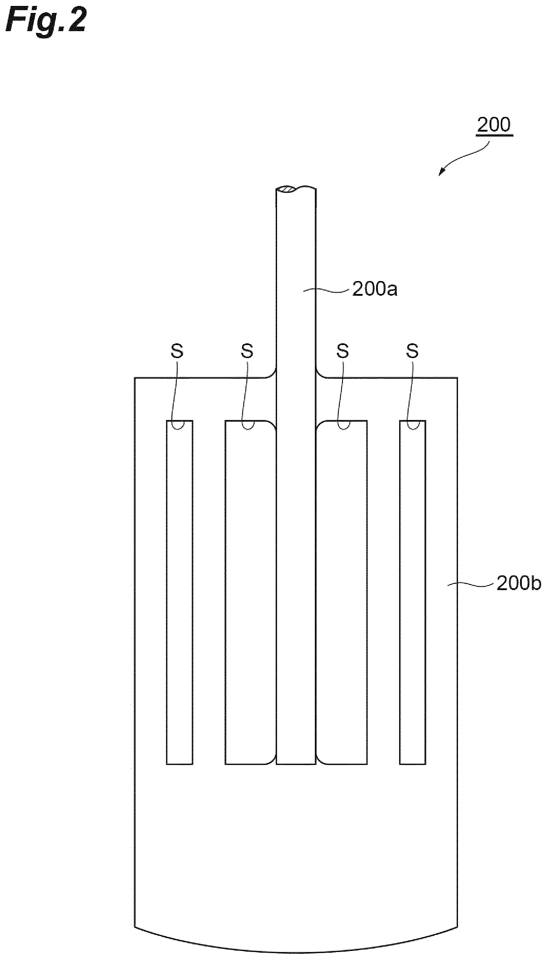

A round-bottomed cylindrical separable flask having an inner diameter of 11 cm and an inner volume of 2L and equipped with a reflux condenser, a dropping funnel, a nitrogen gas inlet tube, and a stirrer was prepared. The stirrer was equipped with stirring blades 200, the outline of which is shown in FIG. 2. The stirring blade 200 includes a shaft 200a and a flat plate portion 200b. The flat plate portion 200b is welded to the shaft 200a and has a curved distal end. Four slits S extending along the axial direction of the shaft 200a are formed in the flat plate portion 200b. The Four slits S are arranged in the width direction of the flat plate portion 200b, the width of the inner two slits S is 1 cm, and the width of the outer two slits S is 0.5 cm. The length of the flat plate portion 200b is about 10 cm, and the width of the flat plate portion 200b is about 6 cm. In the prepared separable flask, 293 g of n-heptane and 0.736 g of a dispersing agent (maleic anhydride-modified ethylene-propylene copolymer, manufactured by Mitsui Chemicals, Inc., High Wax 1105A) were mixed. The mixture in the separable flask was heated to 80°C, while being stirred with a stirrer to dissolve the dispersant in n-heptane. The solution formed was cooled to 50°C.

In a beaker having an inner volume of 300 mL, 92.0 g of a 80.5% by mass acrylic acid aqueous solution (acrylic acid: 1.03 mol) was placed. While cooling from the outside, 147.7 g of a 20.9% by mass aqueous sodium hydroxide solution was added dropwise to the acrylic acid aqueous solution in the beaker to neutralize 75 mol% of acrylic acid. Next, 0.092 g of hydroxy ethyl cellulose (HECAW 15F, manufactured by Sumitomo Seika Chemicals Co., Ltd.), 0.0736 g (0.272 mmol) of potassium persulfate as a water-soluble radical polymerization initiator, and 0.010 g (0.057 mmol) of ethylene glycol diglycidyl ether as an internal crosslinking agent were added and dissolved in the aqueous solution to prepare an aqueous monomer liquid for the first stage.

The obtained aqueous monomer solution was introduced into the separable flask containing the dispersant solution while stirring at a stirrer rotation speed of 425 rpm, and the reaction solution was further stirred for 10 minutes. A surfactant solution in which 0.736 g of sucrose stearate (surfactant, Ryoto Sugar Ester S-370 manufactured by Mitsubishi Chemical Foods Corporation, HLB value: 3) was dissolved in 6.62 g of n-heptane was added to the reaction solution. The inside of the system was sufficiently replaced with nitrogen while stirring at a stirrer rotation speed of 425 rpm. While the stirring was continued, the separable flask was heated in a hot water bath at 70°C for 60 minutes to allow the first stage polymerization reaction to proceed, thereby obtaining a first stage polymerization slurry solution containing the hydrogel-like polymer.

Second stage polymerization

In another beaker having an inner volume of 500 mL, 128.8 g of a 80.5% by mass acrylic acid aqueous solution (acrylic acid: 1.44 mol) was placed. While cooling from the outside, 159.0 g of a 27% by mass aqueous sodium hydroxide solution was added dropwise to the acrylic acid aqueous solution in the beaker to neutralize 75 mol% of acrylic acid. Then, 0.090 g (0.333 mmol) of potassium persulfate as a water-soluble radical polymerization initiator and 0.0116 g (0.067 mmol) of ethylene glycol diglycidyl ether as an internal crosslinking agent were added and dissolved in the aqueous solution to prepare an aqueous monomer solution for the second stage.

The polymerization slurry solution in the first stage was cooled to 25°C, while being stirred at a stirrer rotation speed of 650 rpm. The total amount of the aqueous monomer solution of the second stage was added thereto. After the system was purged with nitrogen for 30 minutes, the separable flask was heated again in a hot water bath at 70°C for 60 minutes while stirring was continued to allow the second stage reaction to proceed.

Removal of water by azeotropic distillation and surface crosslinking

To the reaction solution after the second stage polymerization reaction, 0.589 g of 45% by mass aqueous sodium diethylenetriaminepentaacetic acid was added while stirring the reaction solution. The reaction mixture was heated in an oil bath at 125°C, and 257.6 g of water was removed from the system by azeotropic distillation. Thereafter, 4.42 g of a 2% by mass aqueous solution of ethylene glycol diglycidyl ether (ethylene glycol diglycidyl ether: 0.507 mmol) was added as a surface crosslinking agent, and the reaction solution was maintained at 83°C for 2 hours to promote a surface crosslinking reaction.

Drying and Mixing amorphous silica

The n-heptane was evaporated by heating the reaction solution at 125°C to obtain a dried product of polymer particles. The dried product was passed through a sieve having an opening of 850 $\mu$m, and 0.2% by mass of amorphous silica (Tokuseal NP-S, manufactured by Oriental Silicas Corporation) based on the total mass of the polymer particles was mixed with the polymer particles to obtain 229.8 g of water-absorbing resin particles containing amorphous silica. The median particle size of the water-absorbing resin particles was 362 $\mu$m.

Example 2

The following conditions were applied with respect to the water-soluble radical polymerization agent, the internal crosslinking agent, and the removal of water by azeotropic distillation. Except for these, 232.1 g of water-absorbing resin particles containing amorphous silica were obtained in the same manner as in Example 1. The median particle size of the water-absorbing resin particles was 355 $\mu$m.

First stage polymerization reaction

Water-soluble radical polymerization initiator: 0.092 g (0.339 mmol) of 2,2'-azobis(2-amidinopropane) dihydrochloride and 0.018 g (0.067 mmol) of potassium persulfate
Internal crosslinking agent: 0.046 g (0.026 mmol) of ethylene glycol diglycidyl ether

Second stage polymerization reaction

Water-soluble radical polymerization initiator: 0.129 g (0.476 mmol) of 2,2'-azobis(2-amidinopropane) dihydrochloride and 0.026 g (0.096 mmol) of potassium persulfate.
Internal crosslinking agent: 0.0116 g (0.067 mmol) of ethylene glycol diglycidyl ether

Removal of water by azeotropic distillation

Amount of water removed: 234.6 g

Example 3

The following conditions were applied with respect to the water-soluble radical polymerization agent, the internal crosslinking agent, and the removal of water by azeotropic distillation. Except for these, 231.5 g of water-absorbing resin particles containing amorphous silica were obtained in the same manner as in Example 1. The median particle size of the water-absorbing resin particles was 359 $\mu$m.

First stage polymerization reaction

Water-soluble radical polymerization initiator: 0.0736 g (0.272 mmol) of potassium persulfate

Internal crosslinking agent: 0.010 g (0.057 mmol) of ethylene glycol diglycidyl ether

Second stage polymerization reaction

Water-soluble radical polymerization initiator: 0.090 g (0.333 mmol) of potassium persulfate
Internal crosslinking agent: 0.0116 g (0.067 mmol) of ethylene glycol diglycidyl ether

Removal of water by azeotropic distillation

Amount of water removed: 247.3 g

Example 4

The following conditions were applied with respect to the water-soluble radical polymerization agent, the internal crosslinking agent, and the removal of water by azeotropic distillation. Except for these, 232.0 g of water-absorbing resin particles containing amorphous silica were obtained in the same manner as in Example 1. The median particle size of the water-absorbing resin particles was 359 $\mu$m.

First stage polymerization reaction

Water-soluble radical polymerization initiator: 0.0736 g (0.272 mmol) of potassium persulfate
Internal crosslinking agent: 0.010 g (0.057 mmol) of ethylene glycol diglycidyl ether

Second stage polymerization reaction

Water-soluble radical polymerization initiator: 0.090 g (0.333 mmol) of potassium persulfate
Internal crosslinking agent: 0.0116 g (0.067 mmol) of ethylene glycol diglycidyl ether

Removal of water by azeotropic distillation

Amount of water removed: 271.4 g

Example 5

The following conditions were applied with respect to the water-soluble radical polymerization agent, the internal crosslinking agent, and the removal of water by azeotropic distillation. Except for these, 231.0 g of water-absorbing resin particles containing amorphous silica were obtained in the same manner as in Example 1. The median particle size of the water-absorbing resin particles was 342 $\mu$m.

First stage polymerization reaction

Water-soluble radical polymerization initiator: 0.092 g (0.339 mmol) of 2,2'-azobis(2-amidinopropane) dihydrochloride, and 0.018 g (0.067 mmol) of potassium persulfate
Internal crosslinking agent: 0.0046 g (0.026 mmol) of ethylene glycol diglycidyl ether

Second stage polymerization reaction

Water-soluble radical polymerization initiator: 0.129 g (0.476 mmol) of 2,2'-azobis(2-amidinopropane) dihydrochloride, and 0.026 g (0.096 mmol) of potassium persulfate

Removal of water by azeotropic distillation

Amount of water removed: 224.3 g

Comparative Example 1

The following conditions were applied with respect to the type of stirring blade, the water-soluble radical polymerization agent, the internal crosslinking agent, and the removal of water by azeotropic distillation. Except for these, 224.6 g of water-absorbing resin particles containing amorphous silica were obtained in the same manner as in Example 1. The median particle size of the water-absorbing resin particles was 356 $\mu$m.

First stage polymerization reaction

Stirring blade: 4 inclined paddle blade having a blade diameter of 50 mm surface-treated with a fluororesin (rotation speed: 550 rpm)
Water-soluble radical polymerization initiator: 0.092 g (0.339 mmol) of 2,2'-azobis(2-amidinopropane) dihydrochloride, and 0.018 g (0.067 mmol) of potassium persulfate
Internal crosslinking agent: 0.0046 g (0.026 mmol) of ethylene glycol diglycidyl ether

Second stage polymerization reaction

Stirring blade: 4 inclined paddle bladehaving a blade diameter of 50 mm surface-treated with a fluororesin (rotation speed: 1000 rpm)
Water-soluble radical polymerization initiator: 0.129 g (0.476 mmol) of 2,2'-azobis(2-amidinopropane) dihydrochloride, and 0.026 g (0.096 mmol) of potassium persulfate
Internal crosslinking agent: 0.0116 g (0.067 mmol) of ethylene glycol diglycidyl ether

Removal of water by azeotropic distillation

Amount of water removed: 209.7 g

Comparative Example 2

The following conditions were applied with respect to the type of stirring blade, the water-soluble radical polymerization agent, the internal crosslinking agent, and the removal of water by azeotropic distillation. Except for these, 225.6 g of water-absorbing resin particles containing amorphous silica were obtained in the same manner as in Example 1. The median particle size of the water-absorbing resin particles was 361 $\mu$m.

First stage polymerization reaction

Stirring blade: a stirring blade having 2 stages of 4 inclined paddle blades having a blade diameter of 50 mm surface-treated with a fluororesin (rotation speed: 550 rpm)
Water-soluble radical polymerization initiator: 0.092 g (0.339 mmol) of 2,2'-azobis(2-amidinopropane) dihydrochloride, and 0.018 g (0.067 mmol) of potassium persulfate
Internal crosslinking agent: 0.0046 g (0.026 mmol) of ethylene glycol diglycidyl ether

Second stage polymerization reaction

Stirring blade: 4 inclined paddle blade having a blade diameter of 50 mm surface-treated with a fluororesin (rotation speed: 1000 rpm)

Water-soluble radical polymerization initiator: 0.129 g (0.476 mmol) of 2,2'-azobis(2-amidinopropane) dihydrochloride, and 0.026 g (0.096 mmol) of potassium persulfate
Internal crosslinking agent: 0.0116 g (0.067 mmol) of ethylene glycol diglycidyl ether

Removal of water by azeotropic distillation

Amount of water removed: 207.9 g

Comparative Example 3

A 2L round-bottomed cylindrical separable flask (baffle width: 7 mm) equipped with a reflux condenser, a dropping funnel, a nitrogen gas inlet tube, and a stirrer and having an inner diameter of 110 mm and 4 side wall baffles was prepared. The stirrer was equipped with stirring blades having 2 stages of 4 inclined paddle blades having a blade diameter of 50 mm and surface-treated with a fluororesin. In the prepared separable flask, 660 mL of n-heptane and 0.984 g of sorbitan monolaurate (trade name: Nonionic LP-20R, HLB value: 8.6, manufactured by NOF CORPORATION) were mixed. The mixture in the separable flask was heated at 50°C, while being stirred with a stirrer to dissolve sorbitan monolaurate in n-heptane. The solution formed was cooled to 40°C.
In an Erlenmeyer flask having an internal volume of 500 mL, 92.0 g of a 80.5% by mass acrylic acid aqueous solution (acrylic acid: 1.03 mol) was placed. While cooling with ice from the outside, 146 g of a 21% by mass aqueous sodium hydroxide solution was added dropwise to the aqueous acrylic acid solution in the flask to neutralize 75 mol% of acrylic acid. Then, 0.101 g (0.374 mmol) of potassium persulfate as a water-soluble radical polymerization initiator was added and dissolved in the aqueous solution to prepare an aqueous monomer solution.
The obtained aqueous monomer solution was added to the separable flask containing the solution containing sorbitan monolaurate, and the atmosphere in the system was sufficiently replaced with nitrogen. The reaction solution in the separable flask was kept in a hot water bath at 70°C for 60 minutes while stirring at a stirrer rotation speed of 700 rpm, thereby allowing the polymerization reaction to proceed.
A dispersion of 0.092 g of amorphous silica (Carplex #80, manufactured by Evonik Degussa Japan K. K.) in 100 g of n-heptane was added to the reaction solution containing the hydrogel-like polymer produced by the polymerization reaction, and the reaction solution was stirred for 10 minutes. The separable flask was immersed in an oil bath at 125°C, and 104 g of water was removed from the system by azeotropic distillation. Thereafter, 8.28 g of a 2% by mass aqueous solution of ethylene glycol diglycidyl ether (ethylene glycol diglycidyl ether: 0.95 mmol) was added as a surface crosslinking agent, and the mixture was maintained at an internal temperature of $80 \pm 2$°C for 2 hours to promote a surface crosslinking reaction.
The n-heptane was evaporated by heating the reaction solution at 125°C to obtain a dried product of polymer particles. The dried product was passed through a sieve having an opening of 850 $\mu$m to obtain 90.5 g of water-absorbing resin particles. The median particle size of the water-absorbing resin particles was 420 $\mu$m.

Comparative Example 4

A 2L round-bottomed cylindrical separable flask (baffle width: 7 mm) equipped with a reflux condenser, a dropping funnel, a nitrogen gas inlet tube, and a stirrer and having an inner diameter of 110 mm and 4 side wall baffles was prepared. The stirrer was equipped with stirring blades having 2 stages of 4 inclined paddle blades having a blade diameter of 50 mm and surface-treated with a fluororesin. In the prepared separable flask, 451.4 g of n-heptane and 1.288 g of sorbitan monolaurate (trade name: Nonion LP-20R, HLB value: 8.6, manufactured by NOF CORPORATION) were mixed. The mixture in the separable flask was heated at 50°C, while being stirred with a stirrer to dissolve sorbitan monolaurate in n-heptane. The solution formed was cooled to 40°C.
In an Erlenmeyer flask having an internal volume of 500 mL, 92.0 g of a 80.5% by mass acrylic acid aqueous solution (acrylic acid: 1.03 mol) was placed. While cooling with ice from the outside, 147.7 g of a 20.9% by mass sodium hydroxide aqueous solution was added dropwise to the acrylic acid aqueous solution in the flask to partially neutralize the acrylic acid. Then, 0.1011 g (0.374 mmol) of potassium persulfate as a water-soluble radical polymerization initiator was added and dissolved in the aqueous solution to prepare an aqueous monomer solution.
The obtained aqueous monomer solution was added to the separable flask containing the solution containing sorbitan monolaurate, and the atmosphere in the system was sufficiently replaced with nitrogen. The reaction solution in the separable flask was kept in a hot water bath at 70°C for 60 minutes while stirring at a stirrer rotation speed of 700 rpm, thereby allowing the polymerization reaction to proceed.
A dispersion of 0.092 g of amorphous silica (Tokuseal NP-S, Oriental Silicas Corporation) in 100 g of n-heptane was added to the reaction solution containing the hydrogel-like polymer produced by the polymerization reaction, and the reaction solution was stirred for 10 minutes. The separable flask was immersed in an oil bath at 125°C, and 129.0 g of water was removed from the system by azeotropic distillation. Thereafter, 4.14 g of a 2% by mass aqueous solution of ethylene glycol diglycidyl ether (ethylene glycol diglycidyl ether: 0.475 mmol) was added as a surface crosslinking agent, and the mixture was maintained at an internal temperature of 80 ± 2°C for 2 hours to promote a surface crosslinking reaction.

Water and n-heptane were evaporated by heating the reaction solution at 125°C to obtain a dried product of polymer particles. The dried product was passed through a sieve having an opening of 850 μm to obtain 90.1 g of water-absorbing resin particles. The median particle size of the water-absorbing resin particles was 352 μm.

2. Evaluation

2-1. Water retention capacity

A cotton bag (Cotton broadcloth No. 60, width 100 mm × length 200 mm) containing 2.0g of the water-absorbing resin particles was placed in a 500 mL beaker. Into the cotton bag containing the water-absorbing resin particles, 500 g of a 0.9% by mass sodium chloride aqueous solution (physiological saline) was poured at once so as not to form lumps. The upper part of the cotton bag was tied with a rubber band, and the cotton bag was allowed to stand for 30 minutes to swell the water-absorbing resin particles. The cotton bag after 30 minutes was dehydrated for 1 minutes using a dehydrator (manufactured by Kokusan Co., Ltd., product number: H - 122) set so that the centrifugal force was 167G, and the mass Wa (g) of the cotton bag containing the swollen gel after dehydration was measured. The same operation was performed without adding the water-absorbing resin particles, the empty mass Wb (g) of the cotton bag in a wet state was measured, and the water retention capacity was calculated from the following formula.

$$\text{Water retention capacity (g/g)} = [\text{Wa-Wb}] / 2.0$$

2-2. Water absorption capacity under load

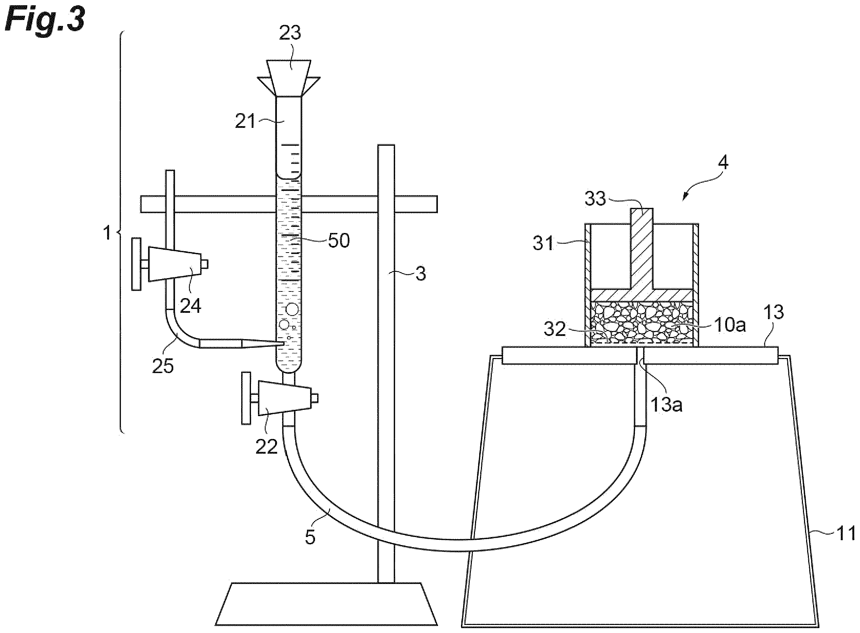

The water absorption amount of the water-absorbing resin particles with respect to physiological saline under a load of 4.14 kPa (water absorption capacity under load) was measured using an apparatus schematically shown in FIG. 3. The water absorption capacity under load was measured 2 times for the one kind of water-absorbing resin particles, and the average value of the measured values was determined. The apparatus of FIG. 3 includes a burette unit 1, a clamp 3, a conduit 5, a stand 11, a measurement table 13, and a measurement unit 4 placed on the measurement table 13. The burette part 1 has a burette tube 21 on which a scale is written, a rubber plug 23 for tightly plugging an opening at the upper part of the burette tube 21, a cock 22 connected to the tip of the lower part of the burette tube 21, and an air introduction tube 25 and a 24 connected to the lower part of the burette tube 21. The bullet part 1 is fixed by a clamp 3. The flat plate-like measurement table 13 has a through hole 13a having a diameter of 2 mm formed in the center thereof, and is supported by a frame 11 having a variable height. The through hole 13a of the measuring table 13 and the 22 of the burette part 1 are connected by a conduit 5. The inner diameter of the conduit 5 is 6 mm.

The measuring part 4 has a cylinder 31 made of acrylic resin, a polyamide mesh 32 bonded to one opening of the cylinder 31, and a weight 33 movable in the vertical direction in the cylinder 31. The cylinder 31 is placed on a measurement table 13 via a polyamide mesh 32. The inner diameter of the cylinder 31 is 20 mm. The opening of the polyamide mesh 32 is 75 μm (200 mesh). The weight 33 has a diameter of 19 mm and a mass of 119.6 g, and can apply a load of 4.14 kPa to the water-absorbing resin particles 10a uniformly arranged on the polyamide mesh 32 as described later.

The measurement of the water absorption capacity under load by the measuring apparatus shown in FIG. 3 was performed in a room at 25°C. First, the 22 and the 24 of the burette section 1 were closed, and 0.9% by mass physiological saline adjusted to 25°C was introduced into the burette tube 21 from the opening in the upper portion of the burette tube 21. Next, the upper opening of the bullet tube 21 was sealed with the rubber stopper 23, and then the 22 and the 24 were opened. The inside of the conduit 5 was filled with 0.9% by mass saline 50 so as not to contain bubbles. The height of the measurement table 13 was adjusted so that the height of the water surface of the 0.9 mass% saline solution reaching the inside of the through hole 13a was the same as the height of the upper surface of the measurement table 13. After the adjustment, the height of the water surface of the 0.9% by mass saline solution 50 in the burette tube 21 was read on the scale of the burette tube 21, and the position was set to 0 point (read value at 0 seconds).

In the measurement section 4, 0.10 g of the water-absorbing resin particles 10a were uniformly arranged on the polyamide mesh 32 in the cylinder 31, the weight 33 was arranged on the water-absorbing resin particles 10a, and the cylinder 31 was installed so that the center portion thereof coincided with the conduit port at the center portion of the measurement table 13. After 60 minutes from the time when the water-absorbing resin particles 10a started to absorb the physiological saline from the conduit 5, the amount of decrease in the physiological saline in the burette tube 21 (I. e., the amount of physiological saline absorbed by the water-absorbing resin particles 10a) Wc (ml) was read, and the physiological saline water absorption capacity of the water-absorbing resin particles 10a under a load of 4.14 kPa was calculated by the following equation. The results are shown in Table 1. Physiological saline water absorption capacity (mL/g) under a load of

$$4.14 \text{ kPa} = \text{Wc (ml)} \text{ / mass (g) of water-absorbing resin particles.}$$

Non-pressurization DW

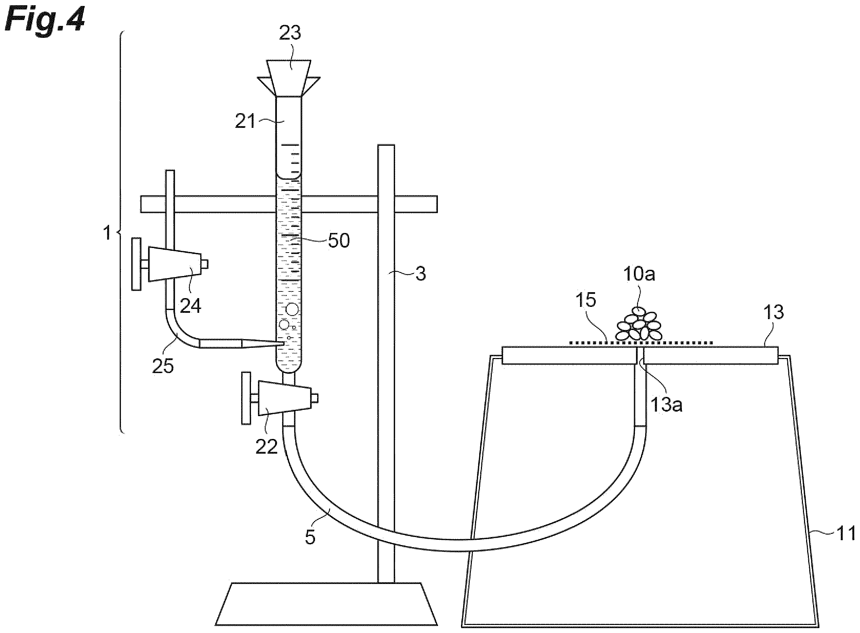

The non-pressurization DW of the water-absorbing resin particles was measured using a measuring apparatus shown in FIG. 4. The measurement was performed 5 times for 1 types of water-absorbing resin particles, and the average value of the measured values at 3 points excluding the lowest value and the highest value was determined.

The measuring apparatus includes a burette portion 1, a conduit 5, a measuring table 13, a nylon mesh sheet 15, a mount 11, and a clamp 3. The burette part 1 has a burette tube 21 on which a scale is written, a rubber plug 23 for tightly plugging an opening at the upper part of the burette tube 21, a cock 22 connected to the tip of the lower part of the burette tube 21, and an air introduction tube 25 and a cock 24 connected to the lower part of the burette tube 21. The bullet part 1 is fixed by a clamp 3. The flat plate-shaper measurement table 13 has a through hole 13a having a diameter of 2 mm formed in the center thereof, and is supported by a frame 11 having a variable height. The through hole 13a of the measuring table 13 and the cock 22 of the burette part 1 are connected by a conduit 5. The inner diameter of the conduit 5 is 6 mm.

The measurement was performed in an environment at a temperature of 25°C and a humidity of 60 ± 10%. First, the cock 22 and the cock 24 of the burette part 1 were closed, and the 0.9% by mass saline 50 adjusted to 25°C was introduced into the burette tube 21 from the opening at the upper part of the burette tube 21. The concentration of 0.9% by mass of the saline solution is based on the mass of the saline solution. After the opening of the bullet tube 21 was sealed with the rubber stopper 23, the cock 22 and the cock 24 were opened. The inside of the conduit 5 was filled with 0.9% by mass saline 50 so as not to contain bubbles. The height of the measurement table 13 was adjusted so that the height of the water surface of the 0.9 mass% saline solution reaching the inside of the through hole 13a was the same as the height of the upper surface of the measurement table 13. After the adjustment, the height of the water surface of the 0.9% by mass saline solution 50 in the burette tube 21 was read on the scale of the burette tube 21, and the position was set to 0 point (read value at 0 seconds).

A nylon mesh sheet 15 (100 mm × 100 mm, 250 mesh, thickness: about 50 μm) was laid in the vicinity of the through-hole 13a on the measurement table 13, and a cylinder having an inner diameter of 30 mm and a height of 20 mm was placed at the center thereof. In this cylinder, 1.00 g of water-absorbing resin particles 10a were uniformly dispersed. Thereafter, the cylinder was carefully removed to obtain a sample in which the water-absorbing resin particles 10a were circularly dispersed in the central portion of the nylon mesh sheet 15. Next, the nylon mesh sheet 15 on which the water-absorbing resin particles 10a were placed was quickly moved to such an extent that the water-absorbing resin particles 10a were not dispersed so that the center thereof was located at the position of the through-hole 13a, and measurement was started. The time when the air bubbles were first introduced into the burette tube 21 from the air introduction tube 25 was defined as the start of water absorption (0 seconds).

The decreased amount of the 0.9% by mass saline 50 in the burette tube 21 (I. e., the amount of the 0.9% by mass saline absorbed by the water-absorbing resin particles 10a) was sequentially read in units of 0.1 mL, and the decreased amount Wc (g) of the 0.9% by mass saline 50 after 10 minutes from the start of water absorption of the water-absorbing resin particles 10a was read. The value of the non-pressurization DW after 10 minutes was determined from Wc by the following equation. The non-pressurization DW is a water absorption amount per 1.00 g of the water-absorbing resin particles 10a.

$$\text{Value of non-pressurization DW after 10 minutes (mL/g)} = \text{Wc / 1.00}$$

2-4. Median particle size

The median particle diameter of the water-absorbing resin particles was measured by the following procedure. That is, a JIS standard sieve was combined with a sieve having a mesh size of 600 μm, a sieve having a mesh size of 500 μm, a sieve having a mesh size of 425 μm, a sieve having a mesh size of 300 μm, a sieve having a mesh size of 250 μm, a sieve having a mesh size of 180 μm, a sieve having a mesh size of 150 μm, and a tray in this order from the top. 50 g of the water-absorbing resin particles were placed in the uppermost sieve of the combination, and classified according to JIS Z 8815 (1994) using a low-tap shaker (manufactured by Iiida Seisakusho Co., Ltd.). After the classification, the ratio of the mass of the particles remaining on each sieve was calculated as a mass percentage with respect to the total amount, and the particle size distribution was determined. The results are shown in Table 100. With respect to the particle size distribution, the ratio of the particles remaining on the sieve in terms of mass percentage (100%) was integrated in the descending order of the particle size, and the relationship between the opening of the sieve and the integrated value of the ratio of the particles remaining on the sieve in terms of mass percentage (100%) was plotted on logarithmic probability paper. By connecting the plots on the probability paper with a straight line, a particle diameter corresponding to an integrated mass percentage of 50% by mass was obtained as a median particle diameter.

2-5. Expansion retention rate

Expansion retention rate was measured in a room at 25 $\pm$ 0.2°C and atmospheric pressure. The same applies to the temperatures of the liquids and instruments used. Into the bottom of a 100 mL measuring cylinder (manufactured by Kartell, 1 scale: 1 mL), 1.000 $\pm$ 0.001 g of the water-absorbing resin particles were uniformly placed. Then, 20.0 $\pm$ 0.1 g of pure water was added all at once. After 10 minutes from the introduction of pure water, the scale of the measuring cylinder corresponding to the position of the highest point of the swollen gel formed by water absorption was read to the value of the decimal first level, and the value was regarded as the volumetric capacity of the swollen gel. The same measurement was performed 3 times, and the volume $V_0$ (mL) of the swollen gel due to absorption of pure water was calculated. The volume $V_1$ of the swollen gel due to absorption of physiological saline was measured by the same measurement using physiological saline (0.9% by mass saline solution) instead of pure water. From the obtained measurement results, the expansion retention rate was calculated by the following formula.

$$\text{Expansion retention rate (\%)} = (V_1 / V_0) \times 100$$

2-6. Absorption amount under pressure of absorber

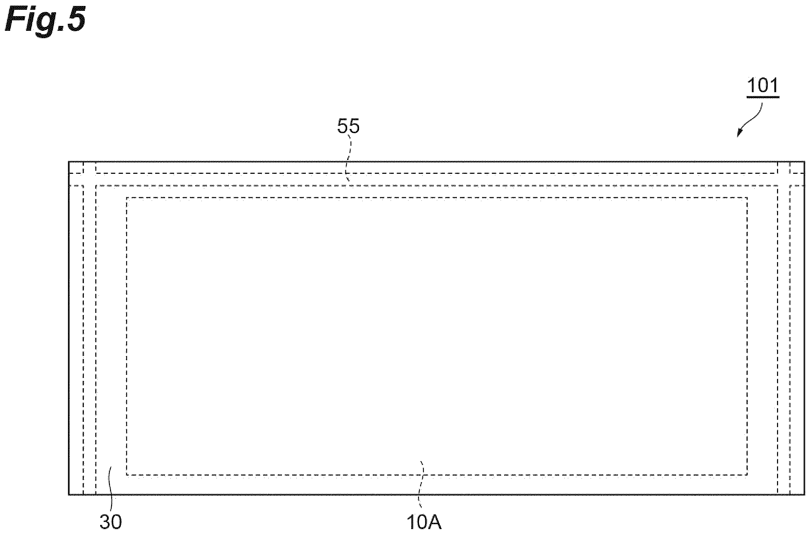

A sheet-shaped absorber having a size of 12 cm $\times$ 40 cm was prepared by uniformly mixing 10 g of the water-absorbing resin particles and 8.0 g of the pulverized pulp by air-papermaking using an air-flow type mixing apparatus (Padformer, manufactured by O-tec Co., Ltd.). The absorber was sandwiched from above and below by 2 sheets of core wrap (tissue paper) having the same size as the absorber and a basis weight of 16 g/m$^2$. The whole was pressurized at a load of 141 kPa for 30 seconds to obtain an absorber with core wrap. A spunbond-meltblown-meltblown-spunbond (SMMS) nonwoven fabric (basis weight 13 g/m$^2$) having a size of 32.5 cm $\times$ 45.0 cm was prepared as the liquid permeable sheet 30, and this was double folded while sandwiching the absorber 10A with the core wrap therebetween as shown in FIG. 5. The end portions 55 along the three sides other than the fold of the double folded liquid permeable sheet 30 were pressure-bonded by a heat sealer to obtain the absorbent article for evaluation 101 comprising the absorber 10A with the core wrap and the liquid permeable sheet 20 wrapping the absorber.

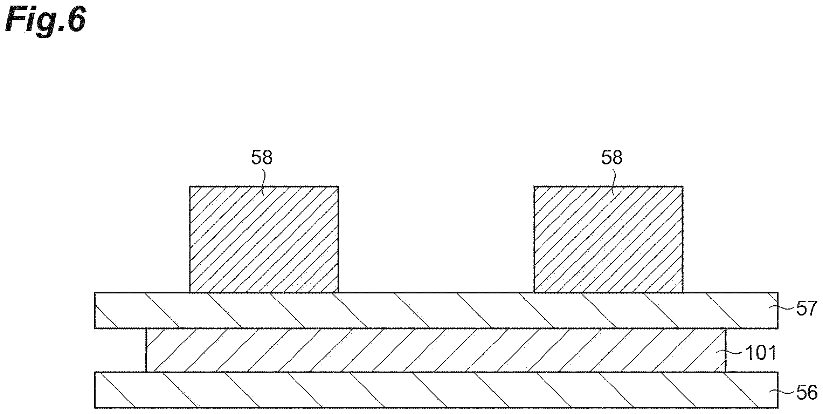

As shown in FIG. 6, the absorbent article for evaluation 101 was sandwiched between 750 g of the lower acrylic resin plate 56 and 1050 g of the upper acrylic resin plate 57. Two weights 58 of 2.5 kg were placed on the upper acrylic resin plate 57 and fixed with tape. The absorbent article for evaluation 101 pressurized by the weight 58 was immersed in physiological saline (0.9% by mass NaCl aqueous solution) at 25 $\pm$ 0.2°C until the upper acrylic resin plate 57 was completely covered with the physiological saline. After the immersion for 15 minutes, the absorbent article for evaluation 101 was removed from the physiological saline. The taken-out absorbent article for evaluation 101 was allowed to drain for 30 seconds by tilting so that the angle between the longitudinal direction of itt and the horizontal plane was 20 degrees, with the weight 58 being placed on the upper acrylic resin plate 57. The mass of the absorbent article for evaluation 101 after draining was measured. The difference between the measured mass of the absorbent article for evaluation 101 after immersion and the mass of the absorbent article for evaluation 101 before immersion was recorded as the absorption amount (g) under pressure of the absorber.

Table 1

| | Water-absorbing resin particles | | | | | | | Absorber |
| --- | --- | --- | --- | --- | --- | --- | --- | --- |
| | Water retention capacity [g/g] | Water absorption capacity under load [mL/g] | DW after 10 min. [mL/g] | Median particle size [μm] | Swollen gen volume [mL] | | Expansion retention rate [%] | Absorption amount under pressure of absorber [g] |
| | | | | | $V_0$ | $V_1$ | | |
| Ex. 1 | 41 | 18 | 60 | 349 | 22.0 | 23.0 | 105 | 540 |

(continued)

| | Water-absorbing resin particles | | | | | | | Absorber |
|---|---|---|---|---|---|---|---|---|
| | Water retention capacity [g/g] | Water absorption capacity under load [mL/g] | DW after 10 min. [mL/g] | Median particle size [$\mu$m] | Swollen gen volume [mL] | | Expansion retention rate [%] | Absorption amount under pressure of absorber [g] |
| | | | | | $V_0$ | $V_1$ | | |
| Ex. 2 | 51 | 17 | 47 | 355 | 21.5 | 21.8 | 101 | 512 |
| Ex. 3 | 35 | 20 | 57 | 357 | 23.5 | 23.3 | 99 | 488 |
| Ex. 4 | 50 | 9 | 65 | 359 | 21.5 | 21.8 | 101 | 537 |
| Ex. 5 | 44 | 24 | 45 | 342 | 22.3 | 23.2 | 100 | 497 |
| Comp Ex. 1 | 28 | 26 | 39 | 356 | 28.5 | 24.3 | 85 | 425 |
| Comp Ex. 2 | 34 | 28 | 45 | 361 | 25.5 | 23.3 | 91 | 466 |
| Comp Ex. 3 | 18 | 21 | 6 | 420 | 43.0 | 25.0 | 58 | 396 |
| Comp Ex. 4 | 38 | 13 | 7 | 369 | 21.9 | 21.2 | 97 | 473 |

As shown in Table 1, it was confirmed that the absorber containing the water-absorbing resin particles exhibiting an expansion retention rate of 98% or more exhibited a sufficiently large absorption amount under pressure.

**Reference Signs List**

1: bullet part, 3: clamp, 4: measurement part, 5: conduit, 10: absorber, 10A: absorber with core wrap, 10a: water-absorbing polymer particles, 10b: fiber layer, 11: frame, 13: measurement table, 13a: through-hole, 15: nylon mesh sheet, 20a, 20b: core wrap, 21: bullet tube, 22: cock, 23: rubber plug, 24: cock, 25: air inlet tube, 30: liquid permeable sheet, 31: cylinder, 32: stirring blade, 200a: shaft, 200b: flat plate portion.

**Claims**

1.   Water-absorbing resin particles,

wherein an expansion retention rate calculated by the formula:

$$\text{expansion retention rate } (\%) = (V_1 / V_0) \times 100, \text{ is 98\% or more,}$$

$V_0$ is a volume of a swollen gel formed when $1.000 \pm 0.001$ g of the water-absorbing resin particles absorb $20.0 \pm 0.1$ g of pure water, and
$V_1$ is a volume of a swollen gel formed when $1.000 \pm 0.001$ g of the water-absorbing resin particles absorb $20.0 \pm 0.1$ g of physiological saline.

2.   The water-absorbing resin particles according to claim 1, wherein the expansion retention rate is 110% or less.

3.   The water-absorbing resin particles according to claim 1 or 2, wherein $V_0$ is 20 to 25 mL.

4.   The water-absorbing resin particles according to any one of claims 1 to 3, wherein a value of non-pressurization DW after 10 minutes is 40 mL/g or more.

5.   An absorbent article comprising:

a liquid impermeable sheet;
an absorber; and
a liquid permeable sheet,
wherein the liquid impermeable sheet, the absorber, and the liquid permeable sheet are disposed in this order, and
the absorber comprises the water-absorbing resin particles according to any one of claims 1 to 4.

6. A method for producing water-absorbing resin particles, the method comprising:

   selecting water-absorbing resin particles in which an expansion retention rate calculated by the formula: expansion retention rate (%) = $(V_1 / V_0) \times 100$ is 98% or more, wherein
   $V_0$ is a volume of a swollen gel formed when $1.000 \pm 0.001$ g of the water-absorbing resin particles absorb $20.0 \pm 0.1$ g of pure water, and
   $V_1$ is a volume of a swollen gel formed when $1.000 \pm 0.001$ g of the water-absorbing resin particles absorb $20.0 \pm 0.1$ g of physiological saline.

7. A method for increasing absorption amount of an absorber containing water-absorbing resin particles under pressure, the method comprising:

   increasing an expansion retention of the water-absorbing resin particles calculated by the formula: expansion retention (%) = $(V_1 / V_0) \times 100$, wherein
   $V_0$ is a volume of a swollen gel formed when $1.000 \pm 0.001$ g of the water-absorbing resin particles absorb $20.0 \pm 0.1$ g of pure water, and $V_1$ is a volume of a swollen gel formed when $1.000 \pm 0.001$ g of the water-absorbing resin particles absorb $20.0 \pm 0.1$ g of physiological saline.

EP 3 936 539 A1

*Fig.1*

# *Fig.2*

**Fig.3**

Fig.4

# Fig.5

EP 3 936 539 A1

**Fig.6**

## INTERNATIONAL SEARCH REPORT

International application No.

PCT/JP2020/009478

### A. CLASSIFICATION OF SUBJECT MATTER
Int.Cl. C08F20/06(2006.01)i, A61F13/53(2006.01)i, B01J20/26(2006.01)i,
B01J20/28(2006.01)i, B01J20/30(2006.01)i
FI: C08F20/06, B01J20/26D, B01J20/28Z, B01J20/30, A61F13/53300
According to International Patent Classification (IPC) or to both national classification and IPC

### B. FIELDS SEARCHED

Minimum documentation searched (classification system followed by classification symbols)
Int.Cl. C08F20/06, A61F13/53, B01J20/26, B01J20/28, B01J20/30

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>A | JP 2000-98198 A (FUJIKURA RUBBER LTD.) 07.04.2000 (2000-04-07), claims, paragraphs [0016], [0059]-[0092], tables 1, 2 | 1, 2, 6<br>3-5, 7 |
| A | JP 2011-19896 A (KAO CORPORATION) 03.02.2011 (2011-02-03), entire text | 1-7 |
| A | JP 2015-521678 A (BASF SE) 30.07.2015 (2015-07-30), entire text | 1-7 |
| A | JP 2018-59124 A (SUMITOMO SEIKA CHEMICALS CO., LTD.) 12.04.2018 (2018-04-12), entire text | 1-7 |
| A | JP 2017-155194 A (SUMITOMO SEIKA CHEMICALS CO., LTD.) 07.09.2017 (2017-09-07), entire text | 1-7 |
| A | WO 2016/148153 A1 (SUMITOMO SEIKA CHEMICALS CO., LTD.) 22.09.2016 (2016-09-22), entire text | 1-7 |

☐ Further documents are listed in the continuation of Box C. ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>13.05.2020 | Date of mailing of the international search report<br>26.05.2020 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT
Information on patent family members

International application No.

PCT/JP2020/009478

```
JP 2000-98198 A    07.04.2000    (Family: none)

JP 2011-19896 A    03.02.2011    CN 102395344 A

JP 2015-521678 A   30.07.2015    US 2015/0119531 A1
                                 entire text
                                 CN 104411732 A

JP 2018-59124 A    12.04.2018    US 2013/0158495 A1
                                 entire text
                                 EP 2615117 A1
                                 TW 201221525 A
                                 CN 103080139 A
                                 KR 10-2013-0140660 A

JP 2017-155194 A   07.09.2017    (Family: none)

WO 2016/148153 A1  22.09.2016    TW 201641609 A
```

Form PCT/ISA/210 (patent family annex) (January 2015)

**EP 3 936 539 A1**

**Patent documents cited in the description**

- JP 2002172139 A
- WO 2011086843 A